# FASCICULE DE BREVET EUROPEEN

(11) **EP 1 187 824 B1**
(45) Date de publication et mention de la délivrance du brevet: **13.11.2002**
(21) Numéro de dépôt: 00915232.3
(22) Date de dépôt: 29.03.2000
(51) Int. Cl.: C07D 333/20, A61K 31/38

(54) **DERIVES DE [(2-SUBSTITUE-5-[3-THIENYL)-BENZYL]-[2-([2-ISOPROPOXY-5-FLUORO]-PHENOXY)-ETHYL]-AMINE, LEUR PROCEDE DE PREPARATION ET LEUR UTILISATION A TITRE DE MEDICAMENTS**
[(2-SUBSTITUIERTE-5-[3-THIENYL)-BENZYL]-[2-([2-ISOPROPOXY-5-FLUORO]-PHENOXY)-ETHYL]-AMINE, VERFAHREN ZU IHRER HERSTELLUNG UND IHRE VERWENDUNG ALS MEDIKAMENTE
NOVEL (2-SUBSTITUTED-5 - 3-THIENYL) -BENZYL]- 2- ( 2-ISOPROPOXY-5-FLUORO] -PHENOXY) -ETHYL] -AMINE DERIVATIVES, METHOD FOR THE PRODUCTION AND USE THEREOF AS MEDICAMENTS

(30) Priorité: 29.03.1999 FR 9903875
(43) Date de publication de la demande: 20.03.2002
(73) Titulaire: PIERRE FABRE MEDICAMENT, 92100 Boulogne (FR)
(72) Inventeur: VACHER, Bernard, F-81100 Castres (FR); CUISIAT, Stéphane, 13, rue du Pasteur Henri Bosc, F-81100 Castres (FR); KOEK, Wouter, F-81290 Viviers les Montagnes (FR)
(74) Mandataire: Ahner, Francis
(86) Numéro de dépôt international: FR0000775
(87) Numéro de publication internationale: WO00058282

(56) Documents cités:
- WO-A-94/20466
- FR-A- 2 248 830
- MEWSHAW R E ET AL: "New generation dopaminergic agents. 2. discovery of 3-OH-phenoxyethylamine and 3-OH-N-phenylpiperazine dopaminergic templates" BIOORGANIC & MEDICINAL CHEMISTRY LETTERS,GB,OXFORD, vol. 8, no. 3, page 295-300 XP004136867 ISSN: 0960-894X cité dans la demande

## Description

La dopamine est un neuromédiateur qui participe au contrôle de la motricité, aux fonctions cognitives, à l'humeur, et intervient sur le circuit de récompense. Cinq types de récepteurs dopaminergiques ont été clonés (D₁-D₅), leurs niveaux d'expression et leurs distributions cérébrales ont été analysé. Parmi ces cinq types de récepteurs, deux types au moins possèdent des isoformes (Proc. Natl. Acad. Sci. USA 1998, 95, 7731). Ces cinq types de récepteurs dopaminergiques bien que pharmacologiquement distincts ont été regroupés en 2 sous-familles : la sous-famille D₁, qui comprend les récepteurs D₁ et D₅ et la sous-famille D₂ qui comprend les récepteurs D₂, D₃ et D₄. Il est possible de différencier l'action pharmacologique des sous-familles D₁ et D₂ mais il est difficile, en général, de différencier la fonction des différents types à l'intérieur de chaque sous-famille.
Un dysfonctionnement de la transmission dopaminergique est impliqué dans la symptomatologie des troubles du système nerveux central tels que les psychoses schizophréniques (Neuropsychopharmacol. 1988, 1, 179), certaines maladies neurodégénératives telle que, par exemple, la maladie de Parkinson (Neurodegenerative Diseases; Jolles, G. ; Stutzmann, J.M.; Eds; Academic Press, 1994, Chap. 8), la dépression (J. Clin. Psychiatry, 1998, 59 (Suppl. 5), 60), la dépendance à certaines substances telles que par exemple la cocaïne, le tabac ou l'alcool (Cell 1997, 90, 991; Nature 1997, 388, 586). Ainsi, par exemple, les antagonistes de récepteurs dopaminergiques centraux du type D₂ constituent une approche classique et cliniquement efficace du traitement des symptômes positifs des psychoses schizophréniques. Cependant, la plupart des composés possédant un tel mécanisme d'action induisent aussi des effets secondaires indésirables tels que des symptômes de type Parkinsonien (Pharmacotherapy 1996, 16, 160) et / ou des désordres neuroendocriniens (Acta Psychiatr. Scand. 1989, 352, 24).

Mewshaw et col. (Bioorg. Med. Chem. Lett. 1998, 8, 295) décrit des phénoxyéthylamines de formule : 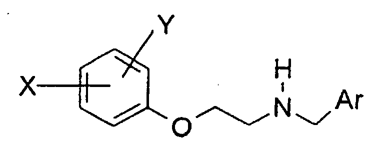 où X représente un atome d'hydrogène, un groupe hydroxyle, un groupe amino ou un groupe méthanesulfonamide, Y représente un atome d'hydrogène ou un atome d'halogène et Ar est un groupe phényl ou 2-thiényl, comme étant des agonistes partiels des récepteur du type D₂.
Les brevets WO 9808817, US 5760070, WO 9808843 et WO 9808819 décrivent respectivement des 4-aminoéthoxyindoles et des 4-aminoéthoxyindolones comme étant des agonistes des récepteurs dopaminergiques du type D₂ ou des inhibiteurs de la synthèse et de la libération de la dopamine.

Unangst et Col. (J. Med. Chem. 1997, 40, 4026) décrit des aryloxyalkylamines de formule : 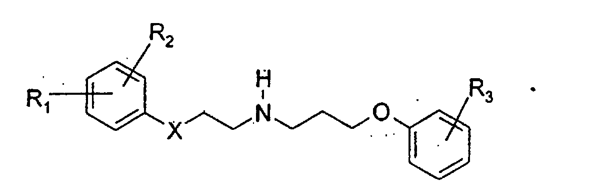 où X représente un atome d'oxygène, de soufre ou un groupe CH₂; R₁ est un atome d'hydrogène, de chlore, un groupe hydroxyle ou hydroxyméthyle, un groupe nitro ou un résidu hydroxycarbonyle; R₂ et R₃ représentent un atome d'hydrogène, un atome d'halogène ou un groupe méthyle. Ces composés sont actifs sur le système dopaminergique, en particulier sur les récepteurs du type D₄ et potentiellement utiles dans le traitement de la schizophrénie.

Le brevet WO 9723482 décrit des octahydropyrrolo[1,2-a]pyrazines de formule : 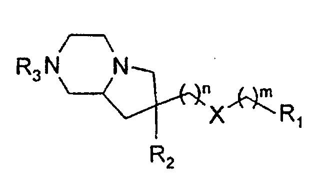 où X représente, entre autres, un atome d'oxygène; m et n = 0, 1, 2 et R₁ est un groupe aromatique non substitué, hétérocyclique ou non, polycyclique ou non. Ces composés ont une affinité pour les récepteurs dopaminergiques, en particulier pour les récepteurs du type D₄.

Les brevets FR 2.702.211, JP 51048627, JP 51052146, DE 2450616 et WO 9631461 décrivent des dérivés de 2-[2-(alkoxy)phénoxy]éthylamines de formule : 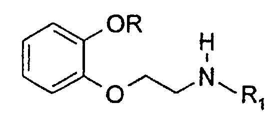 dans laquelle R est un groupe alkyl en C₁-C₄ et R₁ représente une chaîne 4-benzènebutyl, pipéridine-4-méthyl ou 4-benzamidobutyl. Ces composés sont revendiqués comme étant des ligands des récepteurs du sous type 5-HT_{1A} (FR 2.702.211 et WO 9631461) ou des agents hypotenseurs et tranquillisants (JP 51048627, JP 51052146 et DE 2450616). Le brevet EP 707007 décrit des arylamines présentant une double activité : à la fois antagoniste des récepteurs du type D2 et agoniste des récepteurs du sous type 5-HT_{1A} et utiles comme agents antipsychotiques. Le composé EMD-12830 (Drug Data Report 1998, 21) de formule : 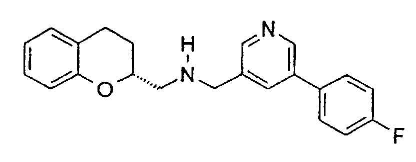 est revendiqué comme un agent antipsychotique atypique (i.e., présentant une moindre propension à provoquer des effets secondaires de type parkinsonien que les antipsychotiques conventionnels).

Le brevet DE 2364685 décrit des phénoxyalkylamines, en particulier la N-[2-(2-méthoxyphénoxy)éthyl]-pyridin-3 ou 4-ylméthanamine sont revendiquées comme des agents hypotenseurs.

Angstein et col. (J. Med. Chem. 1965, 8, 356) décrit des aryloxyalkylamines actives sur le système cardio-vasculaire. Parmi les composés décrits figure la N-[2-(2-méthoxyphénoxy)éthyl]-benzèneméthanamine.

Goldenberg et Col. (Chim. Ther. 1973, 8, 259) décrit, entre autres, des N-[2-(2-méthoxyphénoxy)éthyl-2-benzofuraneméthanamines comme des agents ayant des propriétés vasodilatatrices périphériques.

Le 4-méthoxy-3-[2-[(phénylméthyl)amino]éthoxy]-phénol est décrit dans J. Labelled Compd. Radiopharm. 1993, 33, 1091 et la N-[2-(2-méthoxyphénoxy)éthyl]-furfurylamine est décrite dans FR-1.336.684.

Le brevet WO 9811068 décrit des 1-(2-Pyrimidyl)-4-[(3-aryl)-benzyl]pipérazines comme ligands sélectifs des récepteurs du sous-type D₄.

### Résumé de l'invention :

La présente invention concerne une nouvelle famille de composés qui répondent à la formule générale (1)

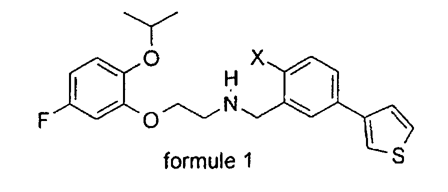

Les composés de cette invention ont une activité antidopaminergique en particulier sur les récepteurs de la sous-famille D₂. A ce titre, les composés de l'invention sont utiles dans le traitement des affections résultant d'une hyperactivité dopaminergique tels que les symptômes schizophréniques et la dépendance à certaines substances. Cependant, l'activité antagoniste des produits de l'invention sur les récepteurs du type D₂, ne s'exerce que lors d'une hyperstimulation dopaminergique transitoire. En l'absence d'une hyperactivité dopaminergique, c'est-à-dire lorsque la concentration en dopamine varie dans des proportions acceptables pour le fonctionnement normal du neurone, les composés de l'invention n'induisent pas une hypoactivité dopaminergique. Les composés de l'invention sont donc utiles dans le traitement des symptômes schizophréniques et présentent l'avantage d'être potentiellement dépourvus des effets secondaires indésirables occasionnés par un blocage excessif des récepteurs du type D₂, tels que les symptômes Parkinsonien et / ou les désordres endocriniens, aux doses thérapeutiquement efficaces pour le traitement des psychoses schizophréniques.
Les composés de l'invention diffèrent donc des dérivés de l'art antérieur par leur formule chimique et leur mécanisme d'action.

### Détails de l'invention :

Plus spécifiquement, la présente invention concerne des composés nouveaux répondant à la formule générale (1). 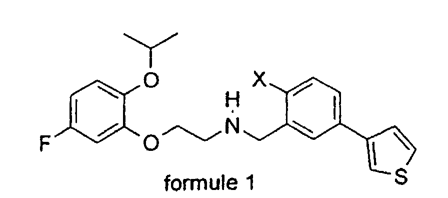 dans laquelle :
X représente :
   - un atome d'hydrogène ou de fluor ;
   - un groupe hydroxy (OH) ou un groupe méthoxy (OCH₃).

L'invention concerne également les sels d'addition et éventuellement les hydrates des sels d'addition des composés de formule générale (1) avec les acides minéraux ou les acides organiques pharmaceutiquement acceptables. L'invention a aussi pour objet les compositions pharmaceutiques contenant à titre de principe actif au moins un des dérivés de formule générale (1) ou un de ses sels ou hydrates de ses sels en combinaison avec un ou plusieurs excipients, adjuvants ou véhicules pharmaceutiquement acceptables. A titre d'exemple on peut citer les complexes d'inclusion, en particulier les complexes d'inclusion formés par les composés de l'invention avec les □- cyclodextrines.

Les compositions pharmaceutiques selon l'invention peuvent être des compositions administrables par voie orale, nasale, sublinguale, rectale ou parentérale. Il est généralement avantageux de formuler de telles compositions pharmaceutiques sous forme de dose unitaire. Chaque dose comprend alors une quantité prédéterminée du principe actif, associée au véhicule, excipients et / ou adjuvants appropriés, calculés pour obtenir un effet thérapeutique donné. A titre d'exemple de forme de dose unitaire administrable par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19è édition, 1995, Mack Publishing Company et peuvent donc être facilement préparées par l'homme de l'art.

Il est connu que la posologie varie d'un individu à l'autre, selon la nature et l'intensité de l'affection, la voie d'administration choisie, le poids, l'âge et le sexe du malade en conséquence les doses efficaces devront être déterminées en fonction de ces paramètres par le spécialiste en la matière. A titre indicatif, les doses efficaces pourraient s'échelonner entre 0,001 et 100 mg/Kg/jour.

Les composés de formule générale (1) peuvent exister sous plusieurs formes tautomères. De telles formes tautomères quoique non explicitement rapportées dans la présente demande pour simplifier la représentation graphique des formules développées sont néanmoins incluses dans le champ d'application de l'invention.

Les composés de formule générale (1) dans lesquels X à la même signification que précédemment, peuvent être préparés selon le procédé décrit dans le schéma A.

### Schéma A

Le composé de formule (1) est préparé par une réaction classique d'amination réductrice entre le composé de formule (2), dans lequel X représente un atome d'hydrogène, de fluor, un groupe hydroxy (OH) ou un groupe méthoxy (OCH3), et l'amine primaire de formule (3). L'expression "une réaction classique d'amination réductrice" signifie que le composé de formule (2) et l'amine (3) sont mis en réaction dans le solvant approprié et que le mélange des réactifs (2) et (3) est ensuite soumis à l'agent réducteur selon une méthode bien connue de l'homme de l'art.
Les composés de formule (1) sont purifiés suivant une ou plusieurs méthodes choisies parmi la cristallisation et / ou les techniques de chromatographie en phase liquide. Ils peuvent être ensuite, si on le désire :
- salifiés au moyen d'un acide pharmaceutiquement acceptable ;
- engagés dans la formation d'un complexe d'inclusion.

L'amine primaire de formule (3) peut-être obtenu par le procédé décrit dans le schéma B.

### Schéma B

La 4-fluoro-2-hydroxy-acétophenone de formule (4) est convertie en l'amine protégée de formule (5) en deux étapes :
- une réaction de Williamson entre le composé de formule (4) et le 1-bromo-2-chloro-ethane conduit à l'éther chloré correspondant (J. Med. Chem. 1989, 32, 105) ;
- substitution de l'atome de chlore au moyen de phtalimide de potassium pour donner le composé de formule (5). Une réaction de Bayer-Villiger, effectuée sur le composé de formule (5) selon Synth. Commun 1989, 11/12, 2001, suivie d'une réaction d'hydrolyse basique du formate intermédiaire conduit au phénol de formule (6). L'alkylation du phénol de formule (6), au moyen du 2-iodo propane, permet d'obtenir l'intermédiaire de formule (7)

L'amine primaire de formule (3), préparée à partir du composé de formule (7) par aminolyse du groupe phtalimido, est utilisée immédiatement dans l'étape suivante d'amination réductrice (Schéma A). La déprotection du groupement amine primaire du composé de formule (7) est effectué par chauffage modéré (60°C) en présence d'un excès d'amino-2-éthanol.

Les aldéhydes de formule (2), dans lesquels dans lesquels X à la même signification que précédemment, sont préparés par le procédé décrit dans le schéma C.

### Schéma C

Le couplage des dérivés bromés de formule (8), disponibles commercialement, avec l'acide 3-thiopheneboronique, disponible commercialement, en présence d'un catalyseur au palladium approprié selon la méthode de Suzuki, conduit directement aux aldéhydes de formule (2).

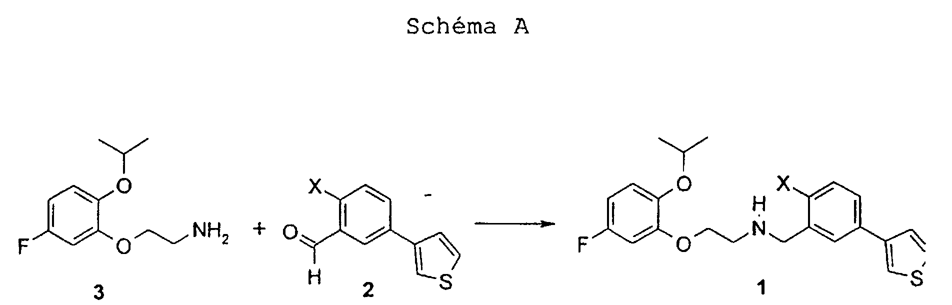

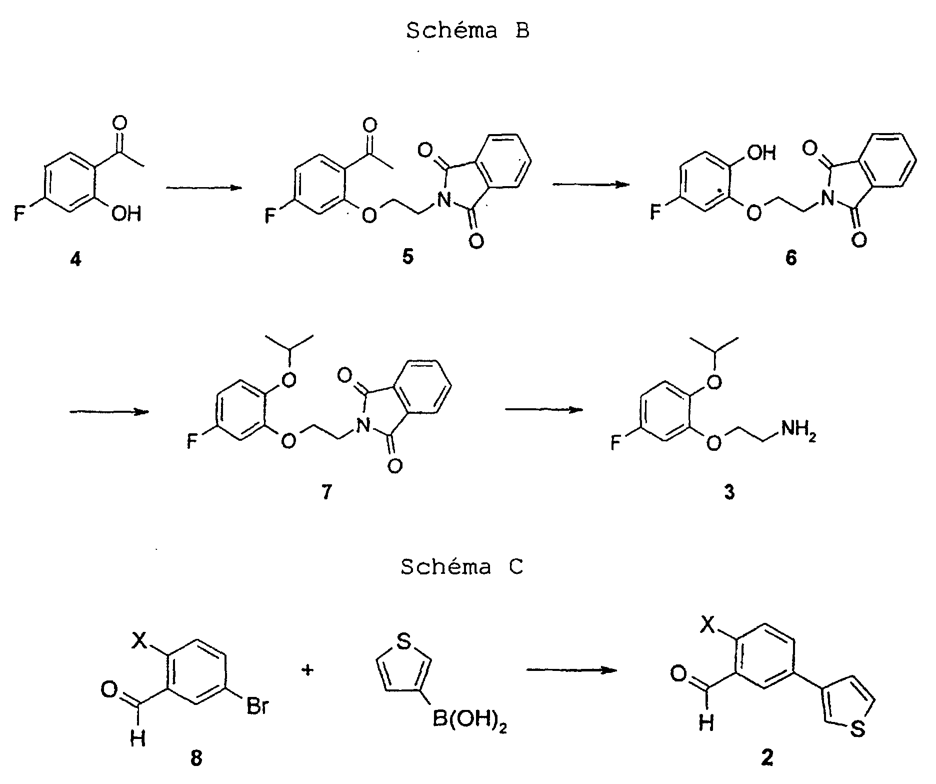

Les exemples suivants illustrent l'invention.
Dans les exemples ci-après :
(i) l'avancement des réactions est suivi par chromatographie couche mince (CCM) et par conséquent les temps de réaction ne sont mentionnés qu'à titre indicatif.
(ii) des formes cristallines différentes peuvent donner des points de fusions différents, les points de fusion rapportés dans la présente demande sont ceux des produits préparés selon la méthode décrite et ne sont pas corrigés.
(iii) la structure des produits obtenus selon l'invention est confirmée par les spectres de résonance magnétique nucléaire (RMN), infrarouge (IR) et l'analyse centésimale, la pureté des produits finaux est vérifiée par CCM.
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par : s, singulet ; d, doublet ; t, triplet ; q, quadruplet ; m, multiplet ; l, large.
(v) les différents symboles des unités ont leur signification habituelle : mg (milligramme) ; g (gramme) ; ml (millilitre) ; °C (degré Celsius) ; mmole (millimole) ; nmole (nanomole) ; cm (centimètre).
(vi) Les abréviations ont la signification suivante : F (point de fusion) ; Eb (point d'ébullition).
(vii) Dans la présente application les pressions sont données en millibars ; par "température ambiante" on entend une température comprise entre 20°C et 25°C.

### 3-(3-Thiényl)-benzaldéhyde (2a).

On ajoute à une solution de 3-bromobenzaldehyde (3 g, 0,016 mole) dans le 1,2-diméthoxy éthane (80 ml) 3,11 g d' acide 3-thiophèneboronique (0,024 mole) suivi d'une solution aqueuse 2 N de carbonate de sodium (5,15 g, 0,048 mole) et d'une quantité catalytique de tetrakis(triphénylphosphine)palladium (0,56 g, 4,9 10-4 mole). Le mélange réactionnel est chauffé à 80°C pendant 16 heures, puis est refroidi à température ambiante et versé dans de l'eau. On extrait par de l'acétate d'éthyle et on lave la phase organique avec une solution aqueuse saturée de chlorure de sodium. La phase organique est séchée sur sulfate de magnésium, filtrée et le solvant est évaporé sous pression réduite. Le produit du titre est isolé par chromatographie sur colonne de silice (éluant : cyclohexane / acétate d'éthyle, 90 / 10). On récupère 2,61 g d'un solide jaune pale.
Rendement : 86%
F : 59°C
1H RMN (CDCl₃) δ : 7.46 (s, 2H); 7.53-7,59 (m, 2H); 7.79 (d, J = 7,68 Hz, 1H) ; 7,86 (d, J = 8,88 Hz, 1H).

### 2-Fluoro-5-(3-thiényl)-benzaldehyde (2b).

On ajoute à une solution de 3-bromo-6-fluoro-benzaldehyde (2 g, 0,016 mole) dans 35 ml de 1,2-diméthoxy éthane 1,89 g d'acide 3-thiopheneboronique (0,015 mole) suivi d'une solution aqueuse 2 N de carbonate de sodium (3,13 g, 0,030 mole) et d'une quantité catalytique de tétrakis(triphénylphosphine)palladium (0,34 g, 2,96 10-4 mole). Le mélange réactionnel est chauffé à 80°C pendant 20 heures, puis est refroidi à température ambiante et versé dans de l'eau. On extrait par de l'acétate d'éthyle et on lave la phase organique avec une solution aqueuse saturée de chlorure de sodium. La phase organique est séchée sur sulfate de magnésium, filtrée et le solvant est évaporé sous pression réduite. Le produit du titre est isolé de l'huile noire obtenue par distillation au four à boules à 150°C sous pression réduite (4.10-2 mmbars). On isole 1,36 g d'une huile jaune pale qui se solidifie.
Rendement : 67%
F : 71°C
1H RMN (CDCl₃) δ: 7,21 (t, J = 9,16 Hz, 1H) ; 7,36-7,43 (m, 2H) ; 7,47 (s, 1H) ; 7,80 (m , 1H) ; 8,06 (dd, J = 6,48 ; 2,24 Hz, 1H) ; 10,37 (s, 1H).

### 2-Hydroxy-5-(3-thiényl)-benzaldéhyde (2c).

On ajoute 0,15 g de catalyseur tétrakis(triphénylphosphine)palladium (1,3 10-4 mole) à une solution de 5-bromo-salicylaldehyde (0,86 g, 0,0043 mole) et d'acide 3-thiophèneboronique (0,6 g, 0,0047 mole) dans 10 ml de 1,2-diméthoxy éthane et 5 ml de méthanol. 1,3 g de fluorure de césium sont ajoutés et le mélange est chauffé à 80°C pendant 20 heures. Le mélange réactionnel est refroidi à température ambiante et versé dans de l'eau. On extrait par du dichlorométhane et on lave la phase organique avec une solution aqueuse saturée de chlorure de sodium. La phase organique est séchée sur sulfate de magnésium, filtrée et concentré sous pression réduite. Le produit du titre est isolé par chromatographie sur colonne de silice (éluant : cyclohexane / acétate d'éthyle, 88 / 12). On récupère 0,62 g d'un solide jaune pale.
Rendement : 71%
F : 120°C
1H RMN (CDCl₃) δ: 7,04 (d, J = 8,32 Hz, 1H); 7.35 (m, 1H); 7.41 (m, 2H); 7,76 (m, 2H); 9,96 (s, 1H) ; 11,02 (s, 1H).

### 2-Méthoxy-5-(3-thiényl)-benzaldéhyde (2d).

3 g de 5-bromo-2-anisaldehyde (0,014 mole) sont dissous dans 45 ml de 1,2-diméthoxy éthane et 2,68 g de d'acide 3-thiopheneboronique (0,021 mole) sont ajoutés, suivi d'une solution aqueuse 2 N de carbonate de sodium (4,44 g, 0,042 mole) et d'une quantité catalytique de tétrakis(triphénylphosphine)palladium (0,48 g, 4,19 10-4 mole). Le mélange réactionnel est chauffé à 80°C pendant 20 heures sous agitation, puis est refroidi à température ambiante et versé dans de l'eau. On extrait par de l'acétate d'éthyle et on lave la phase organique avec une solution aqueuse saturée de chlorure de sodium. On sèche sur sulfate de magnésium puis on filtre et le solvant est évaporé sous pression réduite. Le produit du titre est isolé par chromatographie sur colonne de silice (éluant : cyclohexane / acétate d'éthyle, 94 / 6). Et on isole 2 g d'un solide beige.
Rendement : 67%
F : 79 °C
1H RMN (CDCl₃) δ: 3,97 (s, 3H); 7,02 (d, J = 8,80 Hz, 1H); 7,39 (m, 3H); 7,78 (dd, J = 8,60 ; 2,24 Hz, 1H); 8,05 (d, J = 2,24 Hz, 1H).

### 2-[2-(2-acétyl-5-fluoro-phénoxy)-éthyl]-isoindole-1,3-dione (5).

Etape 1 :1-[2-(2-chloro-éthoxy)-4-fluoro-phényl]-éthanone.

On ajoute à température ambiante 40.5 ml de 1-bromo-2-chloro-éthane (490 mmoles) à une solution de 25 g de 1-[2-hydroxy-4-fluoro-phényl]-éthanone (162 mmoles) dans la 2-butanone (400 ml), suivi de 45 g de carbonate de potassium (320 mmoles) et de 1.26 g d'iodure de potassium (7.59 mmoles).
Le mélange est chauffé à 80°C sous agitation vigoureuse pendant 60 heures, puis est refroidi à température ambiante et versé dans de l'eau glacée. On extrait par de l'acétate d'éthyle et on lave la phase organique par une solution aqueuse saturée de chlorure de sodium. La phase organique est ensuite séchée sur sulfate de magnésium, filtrée et le solvant évaporé. Le produit est cristallisé dans un mélange cyclohexane / acétate d'éthyle. On récupère 15.7 g d'un solide blanc.
Rendement : 45%
F : 67°C
1H RMN (CDCl₃) δ: 2.66 (s, 3H) ; 3.91 (t, 2H) ; 4.32 (t, 2H) ; 6.62 (dd, 1H) ; 6.76 (dt, 1H) ; 7.85 (dt, 1H).

### Etape 2 : 2-[2-(2-acétyl-5-fluoro-phénoxy)-éthyl]-isoindole-1,3-dione (5).

Un mélange de 14.6 g de phtalimide de potassium (79 mmoles) et de 15 g de 1-[2-(2-chloro-éthoxy)-4-fluoro-phényl]-éthanone (69.2 mmoles) dans 150 ml de N,N-diméthylformamide est chauffé à 150°C pendant 6 heures. Le mélange est ensuite refroidi et le solvant est évaporé sous vide. Le solide obtenu est repris dans du dichlorométhane, la phase organique est lavée à l'eau puis avec une solution aqueuse saturée de chlorure de sodium. La phase organique est ensuite séchée sur du sulfate de magnésium, filtrée et le solvant est évaporé.. Le produit obtenu est purifié par cristallisation dans un mélange cyclohexane / acétate d'éthyle. On obtient 18.2 g d'un solide blanc.
Rendement : 76%
F : 140°C
1H RMN (CDCl₃) δ : 2.55 (s, 3H) ; 4.19 (t, 2H) ; 4.34 (t, 2H) ; 6.67 (m, 2H) ; 6.69 (m, 1H) ; 7.77 (m, 2H) ; 7.80 (m, 2H).
IR (KBr) □: 1774, 1716, 1679, 1605, et 1590.

### 2-[2-(5-fluoro-2-hydroxy-phénoxy)-éthyl]-isoindole-1,3-dione (6).

Une solution de 26 g d'acide métachloroperbenzoïque (à 55%, 82.9 mmoles) dans 220 ml de dichlorométhane est agitée pendant une heure puis transférée dans une ampoule de décantation. La phase aqueuse est séparée, la phase organique est placée dans un ballon et refroidie à 0°C. 18 g de 2-[2-(2-acétyl-5-fluoro-phénoxy)-éthyl]-isoindole-1.3-dione (5) (55 mmoles) sont ajoutés par portions et le mélange est agité à température ambiante pendant 16 heures. 6.9 g de bicarbonate de sodium (82 mmoles) sont ensuite introduit par portions et le mélange est agité pendant une heure. Le mélange est ensuite concentré sous vide, 200 ml de méthanol sont ajoutés suivi de 15.2 g de carbonate de potassium (110 mmoles). Le mélange est agité pendant 4 heures à température ambiante, puis le solvant est évaporé, remplacé par 200 ml de dichlorométhane et le mélange est lavé à l'eau et avec une solution aqueuse saturée de chlorure de sodium. La phase aqueuse est séchée sur sulfate de sodium, filtrée et le solvant évaporé. Le produit est cristallisé par du dichlorométhane. On obtient 14 g de produit du titre sous la forme d'un solide blanc.
Rendement : 84%
F : 172°C
1H RMN (DMSO d₆) δ : 3.95 (t, 2H) ; 4.22 (t, 2H) ; 6.58 (dt, 1H) ; 6.75 (t, 1H) ; 6.85 (m, 1H) ; 7.64 (m, 4H) ; 8.77 (s, 1H (échangeable)).

### 2-[2-(5-Fluoro-2-isopropoxy-phénoxy)-éthyl]-isoindole-1,3-dione (7).

On ajoute 7,71 g de carbonate de potassium (56 mmoles) et 7 ml de 2-iodopropane (70 mmoles) à une solution de 14 g de 2-[2-(5-fluoro-2-hydroxy-phénoxy)-éthyl)-isoindole-1,3-dione (6) (47 mmoles) dans 250 ml d'acétonitrile. Le mélange est agité pendant 16 heures à 80°C puis le solvant est évaporé sous pression réduite et le résidu est repris au diéthyle d'ether. La phase organique est lavée avec une solution aqueuse normale de soude puis avec une solution aqueuse saturée de chlorure de sodium. Elle est séchée sur sulfate de magnésium, filtrée et le solvant est évaporé sous vide. Le produit du titre est isolé par chromatographie sur colonne de silice (éluant : cyclohexane / acétate d'éthyle, 82 / 18). On isole 12,2 g d'un solide blanc.
Rendement : 77%
F : 68°C
1H RMN (CDCl₃) δ : 1.20 (s, 3H); 1.21 (s, 3H); 4.14 (t, J = 5,20 Hz, 2H); 4.23 (t, J = 5,20 Hz, 2H) ; 4.30 (sept, J = 6,00 Hz ; 1H) ; 6.56 (dt, 1H) ; 6.65 (dd, 1H) ; 6.79 (dd, 1H); 7.73 (m, 2H) ; 7.86 (m, 2H).

### 2-(5-fluoro-2-isopropoxy)-phénoxy-éthylamine (3).

On ajoute 5 g de 2-[2-(5-fluoro-2-isopropoxy-phénoxy)-éthyl]-isoindole-1,3-dione (7) (14,6 mmoles) à 20 ml d'éthanolamine puis on porte la solution à 60°C pendant 2 heures. Le mélange est versé dans l'eau glacée puis extrait à l'acétate d'éthyle. La phase organique est lavée avec une solution aqueuse saturée en chlorure de sodium, séchée sur sulfate de sodium, filtrée et le solvant évaporé sous vide. On obtient 2,54 g de produit du titre sous la forme d'une huile jaune pâle, utilisé directement dans l'étape suivante sans autre purification.
Rendement : 81%
1H RMN (CDCl₃) δ : 1,31 (s, 3H); 1,32 (s, 3H); 1,46 (brs, 2H) ; 3,09 (t, J = 5,20 Hz, 2H); 3,99 (t, J = 5,20 Hz, 2H); 4,35 (sept, J = 6,00 Hz, 1H); 6,60 (dt, J = 8,32 ; 3,00 Hz 1H); 6,64 (dd, J = 10,16 ; 2,92 Hz, 1H); 6,84 (dd, J = 8,80 ; 5,76 Hz, 1H).

### [3-(3-thiényl)-benzyl]-[2-([5-fluoro-2-isopropoxy-]-phénoxy)-éthyl]-amine (1a).

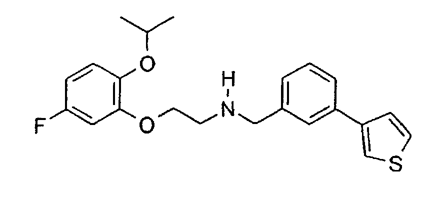

Dans une solution de 3-(3-thiényl)-benzaldéhyde (2a) (700 mg, 3,72 mmoles) et de 2-(5-fluoro-2-isopropoxy)-phénoxy-éthylamine (3) (793 mg, 3,72 mmoles) dans 15 ml de 1,2-dichloroéthane on ajoute 2,5 g de sulfate de magnésium et on chauffe le mélange à 60°C pendant 17 heures sous agitation. On refroidit ensuite la réaction à température ambiante, le solide est filtré et le solvant est évaporé sous pression réduite. On ajoute alors au résidu 15 ml de méthanol et on refroidit à 0°C. On introduit 400 mg de borohydrure de potassium (7,44 mmoles) et on agite le mélange pendant trois heures à 0°C. Le mélange est ensuite versé dans de l'eau glacée, on extrait avec de l'acétate d'éthyle et on lave la phase organique avec une solution aqueuse saturée de chlorure de sodium. On sèche sur sulfate de magnésium puis on filtre et le solvant est évaporé sous pression réduite. Le produit du titre est obtenu par chromatographie sur colonne de silice (éluant : dichlorométhane / méthanol / ammoniaque, 98 /1,5/ 0,5). On isole 970 mg d'une huile incolore.
Rendement : 68%
Préparation du sel : on dissous 0,97 g du produit du titre (2,52 mmoles) dans 10 ml d'éthanol puis on ajoute 0,23 g d'acide oxalique (2,52 mmoles) dans 10 ml d'éthanol. On concentre la solution, le sel précipite et on filtre la solution concentrée. Le sel est séché sous vide à 50°C. On obtient 0,96 g du composé du titre sous forme de sel d'oxalate, poudre cristalline blanche.
F : 189 °C

| Analyse C₂₄H₂₆FNO₆S | | | | |
|---|---|---|---|---|
| Calc % | C 60,62 | H 5,51 | N 2,95 | S 6,74 |
| Tr. | 60,59 | 5,72 | 2,99 | 6,57 |

1H RMN (DMSO d₆) δ : 1,21 (s, 6H); 3,31 (t, J = 4,80 Hz, 2H); 4,32 (m, 4H); 4,48 (sept, J = 6,00 Hz, 1H); 6,73 (m, 1H); 6,99 (m, 2H); 7,28 (m, 2H); 7,55 (d, J = 4,84 Hz, 1H); 7,66 (d, J = 7,04 Hz, 1H); 7,75 (d, J = 7,20 Hz, 1H); 7,88 (d, J = 9,24 Hz, 2H).

IR (KBr) □ : 3436 ; 2979 ; 1718 cm⁻¹.

### [2-Fluoro-5-(3-thiényl)-benzyl]-[2-([5-fluoro-2-isopropoxy]-phénoxy)-éthyl]-amine (1b).

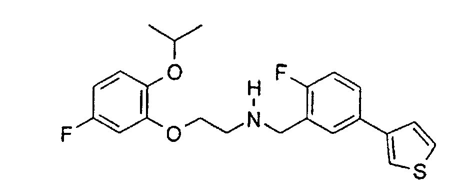

Dans un ballon de 50 ml équipé d'un Dean-Starck on chauffe à 130°C une solution de 2-fluoro-3-(3-thiényl)-benzaldehyde (2b) (350 mg, 1,64 mmoles) et de 2-(5-fluoro-2-isopropoxy)-phénoxy-éthylamine (3) (338 mg, 1,64 mmoles) dans 15 ml de toluène pendant 17 heures. On refroidit ensuite la réaction à température ambiante, le solvant est évaporé sous pression réduite et on dissout le résidu dans 15 ml de méthanol. On refroidit le mélange réactionnel à 0°C et 177 mg de borohydrure de potassium (3,28 mmoles) sont ajoutés. Le mélange est agité pendant sept heures à température ambiante, puis est ensuite versé dans de l'eau glacée, extrait avec de l'acétate d'éthyle et la phase organique est lavée avec une solution aqueuse saturée de chlorure de sodium. On sèche sur sulfate de magnésium, on filtre et le solvant est évaporé sous pression réduite. Le produit du titre est isolé par. chromatographie sur colonne de silice (éluant : dichlorométhane / méthanol/ ammoniaque, 98 /1,5/ 0,5) et on obtient 436 mg d'une huile jaune.
Rendement : 66%
Préparation du sel : on dissous 427 mg du produit du titre (1,06 mmoles) dans 10 ml d'éthanol puis on ajoute 95 mg d'acide oxalique (1,06 mmoles) dans 5 ml d'éthanol. On concentre la solution, le sel précipite et on filtre la solution concentrée. Le sel est séché sous vide à 50°C. On obtient 417 mg du composé du titre sous forme de sel d'oxalate, poudre cristalline blanche.
F : 173 °C

| Analyse C₂₄H₂₅F₂NO₆S | | | |
|---|---|---|---|
| Calc % | C 58,41 | H 5,11 | N 2,84 |
| Tr. | 58,62 | 5,13 | 2,86 |

1H RMN (DMSO d₆) δ : 1,18 (s, 3H); 1,19 (s, 3H); 3,33 (t, J = 4,80 Hz, 2H); 4,27 (t, J = 4,80 Hz, 2H); 4,31 (s, 2H); 4,45 (sept, J = 6,00 Hz, 1H); 6,74 (dt, J = 8,50 ; 2,88 Hz, 1H); 7,00 (m, 2H); 7,32 (t, J = 9,36 Hz, 1H); 7,52 (d, J = 5,00 Hz, 1H); 7,66 (m, 1H); 7,77 (m, 1H); 7,83 (d, J = 1,84 Hz, 1H) ; 7,85 (dd, J = 7,00 ; 1,92 Hz, 1H).
IR (KBr) □ : 3045 ; 2848 ; 1719 cm⁻¹.

### [2-Hydroxy-5-(3-thiényl)-benzyl]-[2-([5-fluoro-2-isopropoxy]-phénoxy)-éthyl]-amine (1c).

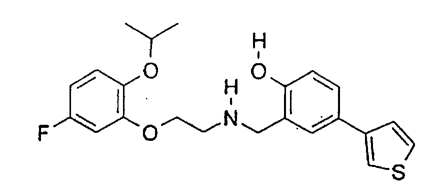

On chauffe une solution de 2-hydroxy-3-(3-thiényl)-benzaldehyde (2c) (400 mg, 1.96 mmoles) et de 2-(5-fluoro-2-isopropoxy)-phénoxy-éthylamine (3) (418 mg, 1,96 mmoles) dans 20 ml de toluène à 130°C pendant 20 heures sous agitation, avec enlèvement continuel de l'eau formée à l' aide d'un Dean-Starck. On refroidit ensuite la réaction à température ambiante, la solution orangée est concentrée sous pression réduite et le résidu est dissout dans 10 ml de méthanol. On refroidit le mélange réactionnel à 0°C et on introduit 211 mg de borohydrure de potassium (3,92 mmoles). Le mélange est agité pendant trois heures à température ambiante, puis est versé dans de l'eau glacée, extrait avec de dichlorométhane et la phase organique est lavée avec une solution aqueuse saturée de chlorure de sodium. On sèche sur sulfate de sodium, on filtre et le solvant est évaporé sous pression réduite. Le produit du titre est isolé par chromatographie sur colonne de silice (éluant : dichlorométhane / méthanol / ammoniaque, 98 /1,5/ 0,5) et on obtient 526 mg d'un solide jaune.
Rendement : 67%
F : 75 °C

Préparation du sel : on dissous 468 mg du produit du titre (1,17 mmoles) dans 10 ml d'éthanol puis on ajoute 105 mg d'acide oxalique (1,17 mmoles) dans 5 ml d'éthanol. On concentre la solution, le sel précipite et on filtre la solution concentrée. Le sel est séché sous vide à 50°C. On obtient 496 mg du composé du titre sous forme de sel d'oxalate, poudre cristalline blanche.
F : 205 °C

| Analyse C₂₄H₂₆FNO₇S | | | |
|---|---|---|---|
| Calc % | C 58,65 | H 5,33 | N 2,85 |
| Tr. | 58,81 | 5,50 | 2,97 |

1H RMN (DMSO d₆) δ : 1,19 (s, 3H); 1,21 (s, 3H); 3,32 (t, J = 4,80 Hz, 2H); 4,29 (m, 4H); 4,47 (sept, J = 6,00 Hz, 1H); 6,75 (dt, J = 8,48 ; 2,76 Hz, 1H); 6,95-7,02 (m, 3H); 7,44 (d, J = 4,96 Hz, 1H); 7,57-7,72 (m, 3H); 7,83 (s, 1H).
IR (KBr) □ : 3450, 3156, 2989, 1733 cm⁻¹.

### [2-Méthoxy-5-(3-thiényl)-benzyl]-[2-([5-fluoro-2-isopropoxy]-phénoxy)-éthyl]-amine (1d).

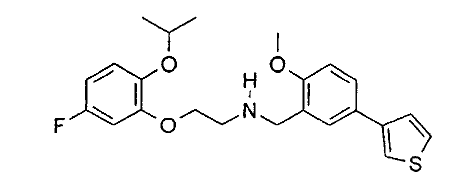

Dans une solution de 2-méthoxy-3-(3-thiényl)-benzaldehyde (2d) (510 mg, 2,34 mmoles) et de 2-(5-fluoro-2-isopropoxy)-phénoxy-éthylamine (3) (500 mg, 2,34 mmoles) dans 10 ml de 1,2-dichloroéthane on ajoute 1,5 g de sulfate de magnésium et on chauffe le mélange à 60°C pendant 20 heures sous agitation. La réaction est refroidit à température ambiante, le solide est filtré et le solvant évaporé sous pression réduite. On dissout le résidu avec 11 ml de méthanol et on refroidit à 0°C. On ajoute 252 mg de borohydrure de potassium (4,68 mmoles) et on agite le mélange pendant 16 heures à température ambiante. Le mélange est ensuite versé dans de l'eau glacée, on extrait avec de l'acétate d'éthyle et on lave la phase organique avec une solution aqueuse saturée de chlorure de sodium. On sèche sur sulfate de sodium puis on filtre et le solvant est évaporé sous pression réduite. Le produit du titre est obtenu par chromatographie sur colonne de silice (éluant : cyclohexane / acétate d'éthyle, 80 / 20). On isole 307 mg d'une huile incolore.
Rendement : 32%
Préparation du sel : on dissous 298 mg du produit du titre (7,17 mmoles) dans 8 ml d'éthanol puis on ajoute 65 mg d'acide oxalique (7,17 mmoles) dans 5 ml d'éthanol. On concentre la solution, le sel précipite et on filtre la solution concentrée. Le sel est séché sous vide à 50°C. On obtient 245 mg du composé du titre sous forme de sel d'oxalate, poudre cristalline blanche.
F : 157 °C

| Analyse C₂₅H₂₈FNO₇S | | | |
|---|---|---|---|
| Calc % | C 59,39 | H 5,58 | N 2,77 |
| Tr. | 59,85 | 5,79 | 3,05 |

1H RMN (DMSO d₆) δ : 1,18 (s, 3H); 1,20 (s, 3H); 3,26 (t, J = 4,80 Hz, 2H); 3,84 (s, 3H); 4,21 (s, 2H); 4,25 (t, J = 4,80 Hz, 2H); 4,45 (sept, J = 6,00 Hz, 1H); 6,75 (dt, J = 8,52 ; 2,92 Hz, 1H); 6,99 (m, 2H); 7,11 (d, J = 8,60 Hz, 1H); 7,49 (d, J = 5,00 Hz, 1H) ; 7,63 (m, 1H); 7,71-7, 94 (m, 3H).
IR (KBr) □ : 2977, 1610 cm⁻¹.

Etude pharmacologique des composés de l'invention.
1- Mesure de l'affinité des composés de l'invention pour les récepteurs D₂.
   L'affinité des composés de l'invention pour les récepteurs du type D₂ a été déterminé par la mesure du déplacement du (³H) YM-09151-2 (NET-1004 70-87 Ci / mmol), selon la méthode décrite dans Naunyn-Schimiedeberg's Arch. Pharmacol. Methods, 1985, 329, 333. Les valeurs de pKi (-log Ki) sont données sous forme de moyenne ± SEM d'au moins 3 expérimentations.
   Le tableau 1 donne, à titre d'exemple, les pKi (D₂) du composé la et de la Rispéridone.
2- Evaluation de l'activité antagoniste des récepteurs D₂ et des effets cataleptogènes des composés de l'invention in vivo.
   Le test mettant en évidence l'activité antidopaminergique in vivo des composés de l'invention repose sur l'inhibition des comportements induit par le méthylphénidate, mesuré chez le rat, selon la méthode décrite dans J. Pharmacol. Exp. Ther. 1993, 267, 181.

Le test permettant d'évaluer la propension des produits de l'invention à provoquer des effets secondaires d'ordre extra-pyramidaux repose sur leur pouvoir cataleptogène, mesuré chez le rat, selon la méthode décrite dans Eur. J. Pharmacol. 1996, 313, 25.
A titre d'exemple, les valeurs obtenues après administration orale du composé 1a sont indiquées dans le tableau 1 en comparaison avec la substance de référence : la Rispéridone.

**Tableau 1**

| Composé | D₂ pKi | Normalisation ED ₅₀ mg / kg | Catalepsy ED₅₀ mg / kg | Index Thérapeutique |
|---|---|---|---|---|
| 1a | 9,45 | 0,7 | > 40 | > 57 |
| Rispéridone | 8,70 | 6,5 | 3,5 | 0,5 |

Il ressort de cette étude que les composés de l'invention possèdent une haute affinité pour les récepteurs du type D₂ ainsi qu'une activité antidopaminergique puissante in vivo. Cependant, de façon surprenante, les composés de l'invention n'induisent pas ou n'induisent qu'à de très fortes doses des effets cataleptogènes comparativementavec la Rispéridone. La Rispéridone est un agent antipsychotique atypique utilisé en clinique (Inpharma® 1998, 1156, 5).
A ce titre, les composés de l'invention qui sont capables de moduler les effets de la dopamine endogène, sont utiles dans le traitement des désordres dopaminergiques tels que la schizophrénie, certaines maladies neurogégénératives et la dépendance à la cocaïne ou à l'alcool ou à des substances analogues.

## Revendications

1. Les composés de formule générale (1) 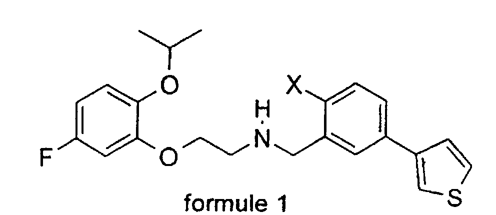 dans laquelle :
X représente :
- un atome d'hydrogène, ou de fluor ;
- un groupe hydroxy (OH) ou un groupe méthoxy (OCH₃).

2. Composés de formule générale (1), selon la revendication 1, **caractérisés en ce qu'**ils sont choisis parmi :
[3-(3-thiényl)-benzyl]-[2-([2-isopropoxy-5-fluoro]-phénoxy)-éthyl]-amine
[2-hydroxy-5-(3-thiényl)-benzyl]-[2-([2-isopropoxy-5-fluoro]-phénoxy)-éthyl]-amine
[2-méthoxy-5-(3-thiényl)-benzyl]-[2-([2-isopropoxy-5-fluoro]-phénoxy)-éthyl]-amine
[2-fluoro-5-(3-thiényl)-benzyl]-[2-([2-isopropoxy-5-fluoro]-phénoxy)-éthyl]-amine.

3. A titre de médicament, les composés de formule (1) selon l'une des revendications 1 à 2.

4. Composition pharmaceutique **caractérisée en ce qu'**elle comprend au moins un composé de formule (1) selon les revendications 1 à 2, ou un sel pharmaceutiquement acceptable d'un composé de formule (1) et un excipient approprié.

5. Utilisation d'un composé de formule (1) ou un sel pharmaceutiquement acceptable d'un composé de formule (1) en quantité suffisante pour la préparation d'un médicament destiné au traitement de la schizophrénie.

6. Utilisation d'un composé de formule (1) ou un sel pharmaceutiquement acceptable d'un composé de formule (1) en quantité suffisante pour la préparation d'un médicament destiné au traitement de la dépendance.

7. Utilisation d'un composé de formule (1) ou un sel pharmaceutiquement acceptable d'un composé de formule (1) en quantité suffisante pour la préparation d'un médicament destiné au traitement des maladies neurodégénératives.

## Claims

1. Compounds of general formula (1) 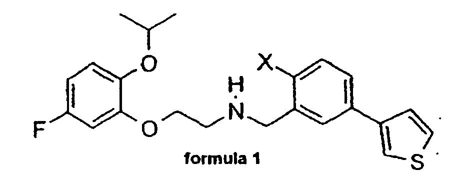 in which:
X represents:
- a hydrogen or fluorine atom;
- a hydroxyl (OH) group or a methoxy (OCH₃) group.

2. Compounds of general formula (1) according to Claim 1, **characterized in that** it is chosen from:
[3-(3-thienyl)benzyl]-[2-([2-isopropoxy-5-fluoro]-phenoxy)ethyl]amine
[2-hydroxy-5-(3-thienyl)benzyl]-[2-([2-isopropoxy-5-fluoro]phenoxy)ethyl]amine
[2-methoxy-5-(3-thienyl)benzyl]-[2-([2-isopropoxy-5-fluoro]phenoxy)ethyl]amine
[2-fluoro-5-(3-thienyl)benzyl]-[2-([2-isopropoxy-5-fluoro]phenoxy)ethyl]amine.

3. Compounds of formula (1) according to either of Claims 1 and 2, as a medicinal product.

4. Pharmaceutical composition, **characterized in that** it comprises at least one compound of formula (1) according to Claims 1 and 2, or a pharmaceutically acceptable salt of a compound of formula (1) and a suitable excipient.

5. Use of a compound of formula (1) or a pharmaceutically acceptable salt of a compound of formula (1) in an amount which is sufficient for the preparation of a medicinal product intended for treating schizophrenia.

6. Use of a compound of formula (1) or a pharmaceutically acceptable salt of a compound of formula (1) in an amount which is sufficient for preparing a medicinal product for treating dependency.

7. Use of a compound of formula (1) or a pharmaceutically acceptable salt of a compound of formula (1) in an amount which is sufficient for preparing a medicinal product for treating neurodegenerative diseases.

## Patentansprüche

1. Verbindungen der allgemeinen Formel (1) 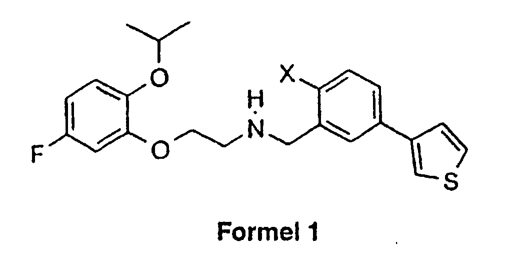 in der X
- ein Wasserstoff- oder Fluoratom;
- eine Hydroxygruppe (OH) oder eine Methoxygruppe (OCH₃) darstellt.

2. Verbindungen der allgemeinen Formel (1) gemäß Anspruch 1, **dadurch gekennzeichnet, dass** sie ausgewählt sind aus:
[3-(3-Thienyl)benzyl]-[2-([2-isopropoxy-5-fluor]phenoxy)ethyl]amin;
[2-Hydroxy-5-(3-thienyl)benzyl]-[2-([2-isopropoxy-5-fluor]phenoxy)ethyl]-amin;
[2-Methoxy-5-(3-thienyl)benzyl]-[2-([2-isopropoxy-5-fluor]phenoxy)ethyl]-amin;
[2-Fluor-5-(3-thienyl)benzyl]-[2-([2-isopropoxy-5-fluor]phenoxy)ethyl]amin.

3. Verbindungen der Formel (1) nach einem der Ansprüche 1 bis 2 als Medikament.

4. Pharmazeutische Zusammensetzung, **dadurch gekennzeichnet, dass** sie mindestens eine Verbindung der Formel (1) nach den Ansprüchen 1 bis 2 oder ein pharmazeutisch annehmbares Salz einer Verbindung der Formel (1) und einen geeigneten Träger umfasst.

5. Verwendung einer Verbindung der Formel (1) oder eines pharmazeutisch annehmbaren Salzes einer Verbindung der Formel (1) in ausreichender Menge für die Herstellung eines Medikaments, das zur Behandlung von Schizophrenie bestimmt ist.

6. Verwendung einer Verbindung der Formel (1) oder eines pharmazeutisch annehmbaren Salzes einer Verbindung der Formel (1) in ausreichender Menge für die Herstellung eines Medikaments, das zur Behandlung von Abhängigkeit bestimmt ist.

7. Verwendung einer Verbindung der Formel (1) oder eines pharmazeutisch annehmbaren Salzes einer Verbindung der Formel (1) in ausreichender Menge für die Herstellung eines Medikaments, das zur Behandlung von neurodegenerativen Erkrankungen bestimmt ist.
